# EUROPEAN PATENT APPLICATION

(11) **EP 4 810 627 A1**
(43) Date of publication of application: **23.09.2026**
(21) Application number: 25445001.8
(22) Date of filing: 18.03.2025
(51) Int. Cl.: C12Q 1/6883

(54) **NEW TARGETS AND BIOMARKERS FOR THE PREVENTION AND TREATMENT OF SKIN AGEING**

(71) Applicant: L'OREAL, 75008 Paris (FR); Pivarcsi, Andor, 169 73 Solna (SE); Pivarcsi Sonkoly, Enikö, 169 73 Solna (SE); Elton, Jonathan, 75 336 Uppsala (SE); Li, Chen, Jinan City, Shandong Province 250011 (CN); Srivastava, Ankit, Palo Alto, CA 94304 (US)
(72) Inventor: Pivarcsi, Andor, SE-169 73 Solna (SE); Pivarcsi Sonkoly, Enikö, SE-169 73 Solna (SE); Elton, Jonathan, SE-75 336 Uppsala (SE); Li, Chen, Jinan City, 250011 (CN); Srivastava, Ankit, Palo Alto, 94304 (US); Bernerd, Françoise, 93601 AULNAY-SOUS-BOIS (FR); Frantz, Marie-Céline, 93601 AULNAY-SOUS-BOIS (FR); Marionnet, Claire, 93601 AULNAY-SOUS-BOIS (FR)
(74) Representative: Fenix Legal KB

(57) **Abstract**

The present invention relates to a method for screening active compounds to prevent and/or treat the signs of skin ageing, said method comprising at least the step of measuring the level of expression of miR-383, and/or PCLAF and/or TTK in a cell or tissue sample and comparing it with a control level.

It further relates to the use of a modulator that inhibits the expression of miR-383; and/or induces, directly or indirectly, the expression of PCLAF and/or TTK prevent and/or treat the signs of skin ageing, to prevent and/or reverse dermal fibroblast senescence in the skin of an individual.

The invention further relates to a method for evaluating the efficacy of a cosmetic treatment against the signs of skin ageing and to an *in vitro* or *ex vivo* method of diagnosing skin ageing.

## Description

### FIELD OF THE INVENTION

The present invention relates to the field of microRNAs (miRNAs) and their role in skin aging and photoaging.

### BACKGROUND OF THE INVENTION

The discovery of non-protein-coding RNAs (ncRNAs) - RNAs that can regulate biological processes but do not code for protein has completely transformed the way we look at gene regulation (MORRIS et al., Nat Rev Genet. 2014;15(6):423-437). The most well-characterized member of ncRNAs are microRNAs (miRNAs), which regulate gene expression at the posttranscriptional level by binding to the 3'UTR regions of mRNAs (FABIAN et al., Annu Rev Biochem. 2010;79:351-379 and BARTEL, Cell. 2018;173(1):20-51). The human genome contains thousands of microRNAs (miRNAs) (OLENA et al., J Cell Physiol. 2010;222(3):540-545). Interestingly, many miRNAs display a highly tissue-restricted expression profile, suggesting roles in the developmental processes and cell-type-specific responses to environmental triggers (CHRISTODOULOU et al., Nature. 2010;463(7284):1084-1088). Evidence derived from mechanistic studies demonstrated key roles for individual miRNAs in diverse cellular processes, for example the regulation of development, stem cell commitment, cell differentiation, proliferation and in the maintenance of cellular and tissue homeostasis (BARTEL and EBERT et al., Cell. 2012;149(3):515-524). The deregulation of miRNA expression has been demonstrated to contribute to diseases (RUPAIMOOLE et al., Nat Rev Drug Discov. 2017;16(3):203-222). A basic characteristic of miRNA-mediated gene regulation is that evolutionarily conserved miRNAs have dozens to hundreds evolutionarily conserved target genes and they act through these gene networks rather than single target gene in order to exert a biological function homeostasis (BARTEL and EBERT et al.). Their importance has made miRNAs attractive tools and targets for novel therapeutic approaches and several miRNA-targeted therapeutics have already reached clinical development (RUPAIMOOLE et al., HAUSSECKER et al., Science. 2015;347(6226):1069-1070, and HAUSSECKER et al., Molecular therapy : the journal of the American Society of Gene Therapy. 2010;18(2):240-242).

MicroRNAs (miRNAs) have recently emerged as important regulators of cellular senescence and aging (SMITH-VIKOS et al., J Cell Sci. 2012;125(Pt 1):7-17 and BOEHM et al., Science. 2005;310(5756):1954-1957). Interestingly, knockdown of the miRNA lin-14 only during adulthood is sufficient to increase longevity in nematodes, indicating that these genes function in adulthood to modulate aging processes.

Skin development is regulated by miRNAs and ablation of miRNAs in the epidermis leads to a lethal phenotype with abnormalities both in the epithelium and epithelial-mesenchymal signaling in mice (YI et al., Cell Death Differ. 2010;17(2):229-235). The inventors and others have shown that miRNAs are deregulated in chronic inflammatory skin diseases in which they regulate apoptosis of T cells, proliferation and differentiation of keratinocytes as well as the communication between epithelial cells and immunocytes in the context of inflammation (XU et al., J Immunol. 2013;190(2):678-688, MEISGEN et al., Exp Dermatol. 2012;21(4):312-314, WEI et al., Eur J Dermatol. 2013, SONKOLY et al., J Allergy Clin Immunol. 2010;126(3):581-589.e581-520, SONKOLY et al., Clin Exp Dermatol. 2008;33(3):312-315, and SONKOLY al., PLoS ONE. 2007;2(7):e610).

Several studies have attempted to investigate the involvement of miRNAs in human skin ageing (MANCINI et al. 2014; MANCINI et al., Aging. 2012;4(11):843-853, RIVETTI DI VAL CERVO et al., Proc Natl Acad Sci U S A. 2012;109(4):1133-1138 and MUTHER et al., Aging. 2017;9(11):2376-2396).

Indeed, among biological processes, skin ageing is a complex phenomenon consisting of two components; intrinsic ageing ("chronological aging"), which is largely genetically determined, and extrinsic ageing caused by environmental factors, primarily solar UV-light ("photoageing") (MANCINI et al., Ageing research reviews. 2014;17:9-15, JENKINS, Mech Ageing Dev. 2002;123(7):801-810, YAAR et al., Br J Dermatol. 2007;157(5):874-887).

Clinically, chronological aging is associated with increased fragility and loss of elasticity of the skin. Histologically, the epidermis and dermis thin with a flattening of the dermo-epidermal junction (GILCHREST, J Am Geriatr Soc. 1982;30(2):139-143). Direct studies with the major cellular constituents of skin, e.g. keratinocytes (in the epidermis) and fibroblasts (in dermis) have demonstrated molecular alterations including altered expression of growth-regulating molecules, decreased matrix synthesis and altered structure of extracellular matrix (ECM) in the dermis with age (JENKINS, Mech Ageing Dev. 2002;123(7):801-810 and PILKINGTON et al., J Invest Dermatol. 2021;141(4s):1087-1095). In photoaged skin, the clinical and biological alterations can be even more pronounced particularly in the dermis, where a complete disorganization of the ECM is observed (BERNEBURG et al, Photodermatol Photoimmunol Photomed. 2000;16(6):239-244).

During the chronological aging, human skin is in particular characterized by decreased cellular proliferation and the accumulation of senescent cells, which limit longevity. Cellular senescence contributes to skin aging by impeding tissue function and promoting age-related dysfunction. UV exposure, especially UVA, accelerates senescence in dermal fibroblasts, leading to their dysfunction and secretion of the Senescence-Associated Secretory Phenotype (SASP) - a mix of pro-inflammatory cytokines, growth factors, and proteases (FITSIOU et al., J Invest Dermatol. 2021; 141(4s): 1119-1126 2021; and WANG et al., Front Genet. 2018;9:247).

Senescent fibroblasts exhibit key hallmarks of skin ageing such as the accumulation of p16INK4A, CDKN1A, and β-galactosidase, along with chromatin changes and metabolic alterations, contributing to the decline in skin function. Additionally, epigenetic modifications, another key hallmark of skin ageing, include alterations in histone methylation (H3K9me3, H3K27me3) and upregulation of transposable elements and further play critical roles in cellular senescence (LÓPEZ-OTÍN et al, Cell. 2023;186(2):243-278).

Sirtuin 1 (SIRT1), an NAD+-dependent deacetylase, is another key regulator in skin aging. Its expression decreases with age and fibroblast passage, impairing DNA repair, oxidative stress response, and extracellular matrix renewal (BIELACH-BAZYLUK et al., Cells. 2021;10(4); and KIM et al., Microsc Res Tech. 2015;78(4):277-282). Studies suggest that SIRT1 activation may counteract photoaging and prevent premature senescence, highlighting its importance in skin longevity (LEE et al., Sirtuin signaling in cellular senescence and aging. BMB Rep. 2019;52(1):24-34). Senescent fibroblasts also enhance matrix degradation through the production of matrix metalloproteinases (MMPs) and pro-inflammatory cytokines, further exacerbating skin aging (WALDERA LUPA et al., J Invest Dermatol. 2015;135(8):1954-1968).

Thus, skin aging is marked by the accumulation of senescent fibroblasts, epigenetic modifications, and senescence-associated secretory phenotype (SASP), all of which contribute to tissue degradation and diminished regenerative capacity, offering valuable insights for developing therapeutic strategies to promote skin longevity.

However, still relatively little is known about the involvement of miRNAs in the aging of skin. Indeed, while genetic alterations and protein-coding genes have been extensively studied in the context of ageing, little is understood about the contribution of miRNAs in the process.

To identify ageing-associated miRNAs, the inventors recently performed the systematic investigation of differentially expressed miRNAs during human intrinsic and extrinsic ageing. The global analysis of miRNA expression in skin samples collected from sun-exposed and non-sun-exposed areas of the arm of volunteers of different age groups identified a set of differentially expressed miRNAs between sun-protected and sun-exposed skin. Among them, the inventors validated the altered expression of several of them by qRT-PCR: miR-383, miR-145 and miR-34a were found up-regulated while miR-6879, miR-3648 and miR-663b were found downregulated in sun-exposed skin compared with sun-protected skin samples. One of the most interesting miRNAs whose expression was altered during skin ageing and during photoaging was miR-383, which was differentially expressed between age groups irrespective of sun exposure conditions, suggesting a synergetic effect of intrinsic and extrinsic aging on their expression (SRIVASTAVA et al., Scientific reports. 2018;8(1):12990). Thus, among others, miR-383 is a biomarker of skin ageing and photoaging.

However, a biomarker does not necessarily constitute the cause of the observed phenotype as shown by NISSAN et al. The authors indeed identified five microRNAs that were specifically overexpressed during the early stages of the differentiation process of keratinocytes (miR-200a, miR-200b, miR-203, miR-205 and miR-429). In order to know if each miRNA was a cause or a consequence of the differentiation process in keratinocytes, they performed gain and loss of function experiment for each miRNA. For one miRNA (miR-203), the functional experiments led to a hESC phenotype change towards differentiation, showing that miR-203 is an actor in this process. For the four other miRNAs, no phenotype change was observed in functional experiments, illustrating that they would only be consequence and not cause of the differentiation process (NISSAN et al., Dev Biol. 2011;356(2):506-515).

Furthermore, several direct target mRNAs of miR-383 have been described in the literature. Among them, PCLAF (PCNA-asssociated Factor, also named KIAA0101) was identified in esophageal squamous cell carcinoma as a direct target of miR-383 (SUN et al., Thorac Cancer. 2022;13(12):1751-1762). In skin, no link has been described between PCLAF and miR-383. Known as regulator of DNA repair during DNA repair, PCLAF was also shown to be downregulated in genotoxic stress (etoposide)-induced senescence in immortalized human lung fetal fibroblasts (WI38-ETO), and in RAS-induced senescence in human lung fibroblasts (IMR90-RAS) (COLLIN et al., Cell death & disease. 2018;9(3):259). In addition, repression of PCLAF by miR-197-p inhibits sarcomagenesis and induces cellular senescence (JAIN et al., Mol Carcinog. 2019;58(8):1376-1388). To date there was no link between PCLAF and skin senescence or skin aging or skin photoaging.

Among proteins involved in senescence, TTK (Dual Specificity protein kinase also known as Mps1) is a known critical mitotic checkpoint protein during chromosome segregation, in mitosis. It was also described as an inducer of senescence and autophagy in liver cancer (MIAO et al., Scientific reports. 2016;6:33121). To date there was no link between TTK and skin senescence or skin aging or skin photoaging.

Therefore, there remains a need to identify new targets, in particular new miRNA targets, that can be used to prevent and/or treat the signs of skin chronological aging and photoageing in individuals.

There further remains a need to identify new targets, in particular new miRNA targets, to prevent and/or reverse dermal fibroblast senescence in the skin of an individual.

There also remains a need to identify new biomarkers of skin chronological ageing and photoageing. In particular, there remains a need to identify new biomarkers that may be used to evaluate the efficacy of a cosmetic treatment against the signs of skin ageing.

Finally, there remains a need to identify new biomarkers that may be used in an in *vitro* or *ex vivo* method of diagnosing skin ageing in an individual.

The present invention aims to address one or more of the above-mentioned problems.

### SUMMARY OF THE INVENTION

The inventors have shown that, in human skin, miR-383 was mostly expressed in dermal fibroblasts and that its overexpression induced senescence in fibroblasts.

In addition, decreased endogenous expression of miR-383 (by knockdown via CRISPR Cas9 technology) enabled reducing/reversing the senescent phenotype of dermal fibroblasts.

The inventors have further shown that PCLAF is a direct target of miR-383 in skin and that reducing its expression by knockdown via CRISPR Cas9 technology enabled reducing or even reversing the senescent phenotype of dermal fibroblasts. Finally, they have shown that TTK is another target of miR-383.

As such, a first object of the present invention relates to a method for screening active compounds to prevent and/or treat the signs of skin ageing, said method comprising the following steps:
(i) contacting a candidate active compound with a cell or tissue sample, in particular a skin cell or skin tissue sample;
(ii) measuring the level of expression of miR-383, and/or PCLAF and/or TTK in the cell or tissue sample, in particular the skin cell or skin tissue sample;
(iii) comparing the level of expression of miR-383, and/or PCLAF and/or TTK measured in step (ii) with a control level; and
(iv) selecting the candidate active compound as an active compound to prevent and/or treat the signs of skin ageing when:
   a. the level of expression of miR-383 measured in step (ii) is lower than the control level, and/or
   b. the level of expression of PCLAF measured in step (ii) is higher than the control level, and/or
   c. the level of expression of TTK measured in step (ii) is higher than the control level.

In a particular embodiment,
- measuring the level of expression of PCLAF comprises measuring the mRNA levels of PCLAF and/or measuring the protein levels of PCLAF; and/or
- measuring the expression of TTK comprises measuring the mRNA levels of TTK and/or measuring the protein levels of TTK.

In particular, the control level in step (iv) a. is the level expression of miR-383 measured in the cell or tissue sample, in particular the skin cell or skin tissue sample, before step (i); the control level in step (iv) b. is the level of expression of PCLAF measured in the cell or tissue sample, in particular the skin cell or skin tissue sample, before step (i) and/or the control level in step (iv) c. is the level of expression of TTK measured in the cell or tissue sample, in particular the skin cell or skin tissue sample, before step (i).

In particular, the method is implemented *in vitro* or *ex vivo.*

According to another object, the present invention relates to a use of at least one modulator that:
(a) inhibits, directly or indirectly, the expression of miR-383; and/or
(b) induces, directly or indirectly, the expression of PCLAF and/or TTK;
to prevent and/or treat the signs of skin ageing.

The invention further relates to a use of at least one modulator that:
(a) inhibits, directly or indirectly, the expression of miR-383; and/or
(b) induces, directly or indirectly, the expression of PCLAF and/or TTK;
to prevent and/or reverse dermal fibroblast senescence in the skin of an individual.

In a particular embodiment, the at least one modulator that inhibits directly or indirectly the expression of miR-383 is an antisense oligonucleotide, in particular is an antisense oligonucleotide selected from the list consisting of antagomirs such as antisense microRNA oligonucleotides (AMO), and shRNAs.

In particular, the antisense oligonucleotide comprises a nucleic acid sequence:
- having at least 80 %, or at least 85 %, or at least 90% or at least 95 % identity sequence with the nucleic acid sequence set forth as SEQ ID NO: 1 and comprising at least a sequence having 100% sequence identity with the nucleic acid sequence set forth as SEQ ID NO: 3, or
- having at least 80 %, or at least 85 %, or at least 90% or at least 95 % identity sequence with the nucleic acid sequence set forth as SEQ ID NO: 2 and comprising at least a sequence having 100% sequence identity with the nucleic acid sequence set forth as SEQ ID NO: 4,
or a derivative thereof; or solvates thereof such as hydrates; or optical and geometric isomers and tautomers thereof; or organic or mineral base or acid salts thereof..

In a particular embodiment, the antisense oligonucleotide comprises a sequence having 100% sequence identity with the nucleic acid sequence set forth as SEQ ID NO: 1 or comprises a sequence having 100% sequence identity with the nucleic acid sequence set forth as SEQ ID NO: 2, or a derivative thereof; or a solvate thereof such as a hydrate; or an optical or geometric isomer or tautomer thereof; or an organic or mineral base or acid salt thereof, and in particular consists of the nucleic acid sequence set forth as SEQ ID NO: 1 or of the nucleic acid sequence set forth as SEQ ID NO: 2.

In particular, the modulator that induces directly or indirectly the expression of PCLAF is selected from the list consisting of Tosylarginine methyl ester (TAME); proTAME; and Cepafungin I; as well as solvates thereof such as hydrates; optical and geometric isomers; tautomers thereof; and organic or mineral base or acid salts thereof.

A further aspect of the present invention relates to a method for evaluating the efficacy of a cosmetic treatment against the signs of skin ageing comprising at least the steps of :
1) applying the cosmetic treatment on the skin of an individual;
2) measuring the level of expression of PCLAF and/or the level of expression of TTK in a cell or tissue, in particular a skin cell or skin tissue sample, sample of the individual;
3) comparing the level of expression of PCLAF and/or the level of expression of TTK measured in step 2) with a control level.

In a particular embodiment, the method further comprises the additional step of:
4) after step 3), determining that the cosmetic treatment is efficient against the signs of skin ageing if the level of expression of PCLAF and/or the level of expression of TTK measured in step 2) is/are higher than the control level(s).

In a particular embodiment of the methods or uses, the signs of skin ageing are selected from the group consisting of wrinkles; fine lines; loss of elasticity; sagging of the skin, marked microrelief of the skin, the appearance of roughness, thinning of the skin, loss of firmness; loss of radiance; and pigmentation disorders such as pigmentation spots including actinic lentigo and melasma.

According to another aspect, the present invention relates to an *in vitro* or *ex vivo* method of diagnosing skin ageing in an individual comprising at least the step of measuring the level of expression of PCLAF and/or the level of expression of TTK in a cell or tissue sample, in particular a skin cell or skin tissue sample, of said individual, in particular in dermal cells, more particularly in dermal fibroblasts.

Finally, the present invention relates to a method according to the invention or the *in vitro* or *ex vivo* method of the invention, wherein measuring the level of expression of PCLAF includes measuring the mRNA levels of PCLAF and/or the protein levels of PCLAF and/or wherein measuring the level of expression of TTK includes measuring the mRNA levels of TTK and/or the protein levels of TTK.

### BRIEF DESCRIPTION OF THE FIGURES

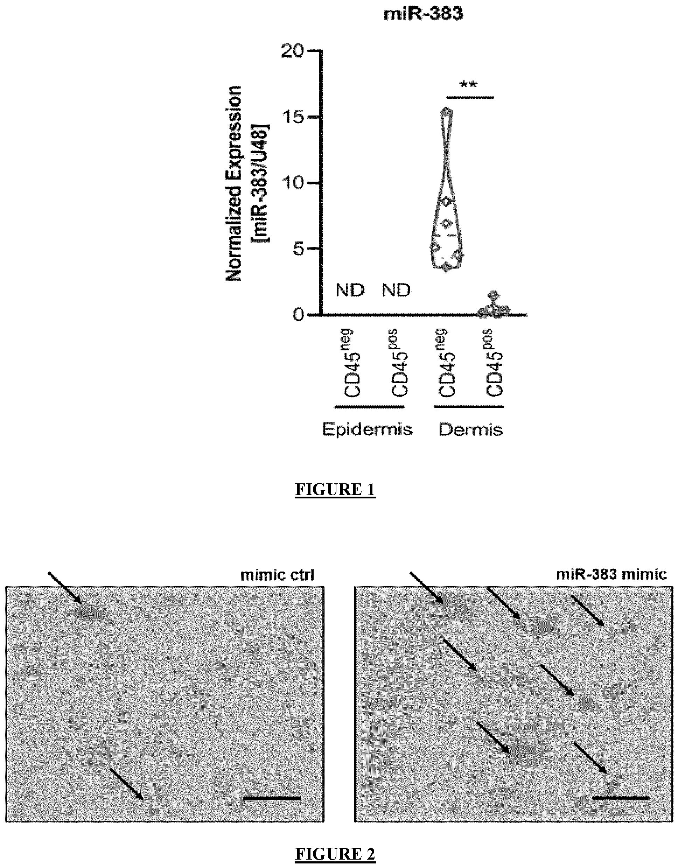

**Figure 1** shows the variation of the normalized expression of [miR-383/U48] in CD45-positive (CD45^{pos}) and CD45-negative (CD45^{neg}) cells from the epidermis (left side of the graph) and from the dermis (right side of the graph). miR-383 expression was normalized against U48 expression. (** p=0,0043, Mann-Whitney U test)
**Figure 2** shows microscope observations of human primary dermal fibroblasts transfected with miR-383 mimic (left image) or with mimic control (right image). Arrows indicate examples of cells with β-Galactosidase staining.

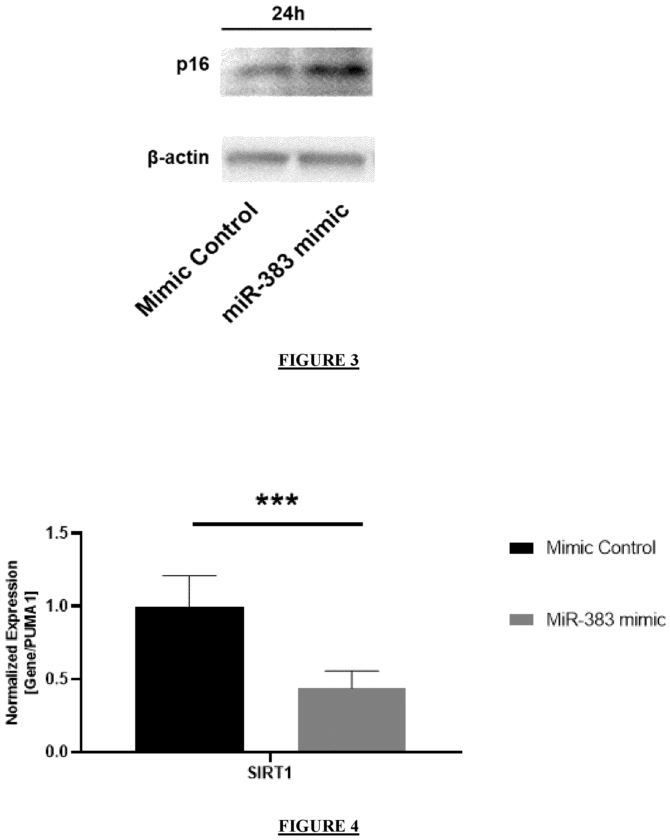

**Figure 3** shows the Western Blot results of p16 and β-actin level expressions in human primary dermal fibroblasts transfected with miR-383 mimic (right column) or with mimic control (left column). β-actin level was used as a control to ensure the equivalent loading and transfer of total cellular protein.
**Figure 4** shows the normalized expression of [SIRT1/PUMA1] in human primary dermal fibroblasts transfected with miR-383 mimic (right column) or with mimic control (left column). SIRT1 mRNA expression was normalized against PUM1 expression. (*** p=0,0002, Student's t-test)

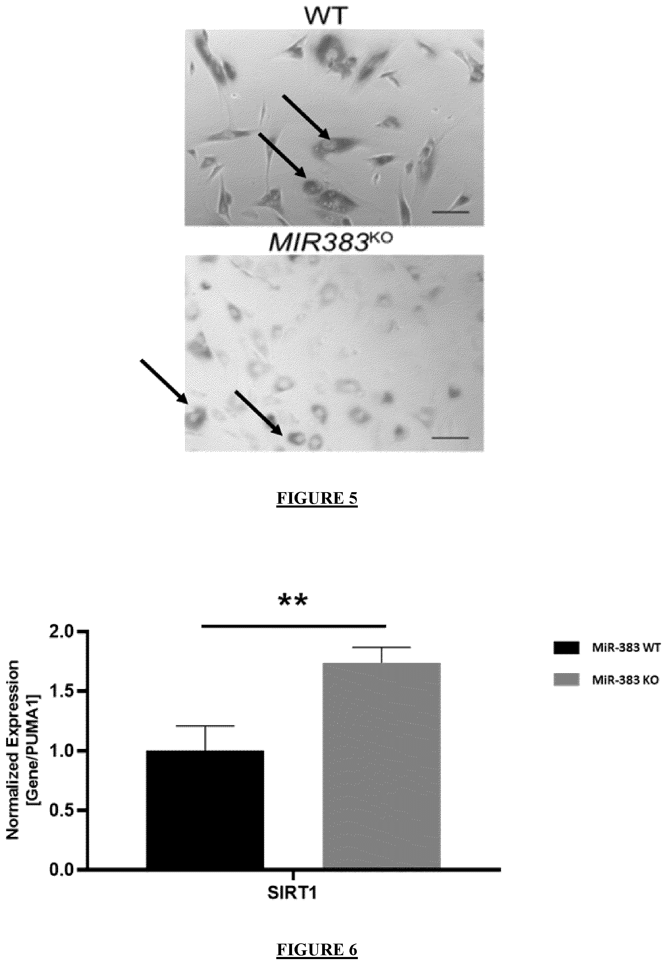

**Figure 5** shows microscope observations of WT (Wild Type) human dermal fibroblasts (top image) or miR-383-deleted human dermal fibroblasts (MIR383^{KO}) (bottom image). Arrows indicate example of cells with β-Galactosidase staining.
**Figure 6** shows the normalized expression of [SIRT1/PUMA1] in WT human primary dermal fibroblasts (miR-383 WT) (left column) or in human primary dermal fibroblasts wherein miR-383 gene was ablated (miR-383 KO) (right column). SIRT1 mRNA expression was normalized against PUM1 expression. (** p<0,01, Student's t-test)

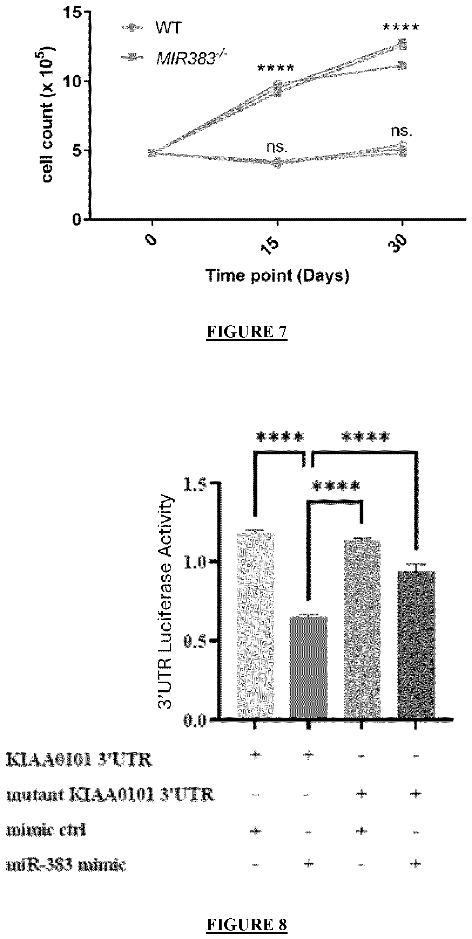

**Figure 7** shows the variation in cell count of the WT human dermal fibroblasts (WT) (● curve) or of the human dermal fibroblasts in which MIR383 gene was deleted (MIR383-/-) (**■** curve) over time (in days). (**** p<0,0001, Two-way ANOVA)
**Figure 8** shows the variation in 3'UTR luciferase activity in fibroblasts transfected with luciferase reporter vectors comprising WT 3' UTR of KIAA0101/PCLAF mRNA co-transfected with mimic control (first column from the left to right), comprising WT 3' UTR of KIAA0101/PCLAF mRNA co-transfected with miR-383 mimic (second column from left to right), comprising mutated 3' UTR of KIAA0101/PCLAF mRNA co-transfected with mimic control (third column from left to right) or comprising mutated 3' UTR of KIAA0101/PCLAF mRNA co-transfected with miR-383 mimic (fourth column from left to right). (**** p<0,0001 One-way ANOVA)

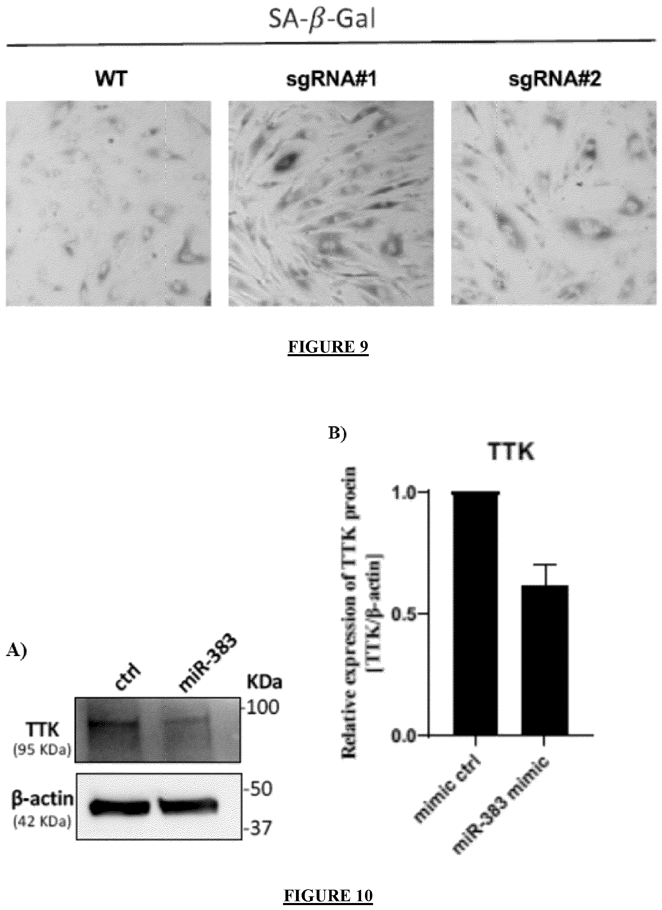

**Figure 9** shows microscope observations of WT human dermal fibroblasts (image on the left), of human fibroblasts wherein genetic ablation of KIAA0101/PCLAF has occurred with sgRNA#1 (middle image) and of human fibroblasts wherein genetic ablation of KIAA0101/PCLAF has occurred with sgRNA#2 (image on the right) on β-Galactosidase staining.
**Figure 10** shows the Western Blot results of TTK1 protein expression in human primary dermal fibroblast transfected using mimic control (left migration lane) or transfected using miR-383 mimic (right migration lane). β-actin level was used as a control to ensure the equivalent loading and transfer of total cellular protein (**Figure 10A**). It further shows the relative expression of TTK protein in human primary dermal fibroblast transfected using mimic control (left column) or transfected using miR-383 mimic (right column). TTK expression was normalized against β-actin expression (**Figure 10B**).

### DETAILED DESCRIPTION OF THE INVENTION

As used throughout the specification, *"PCLAF"* refers to PCNA-associated Factor.

As used throughout the specification, *"TTK"* refers to Dual Specificity protein kinase, and is also known as and referred to as Mps1

In the context of the present invention, the terms *"treat"* or *"treatment",* in particular when used in relation with a condition, denotes slowing down or stopping the development or progression of the condition, causing regression of the associated clinical symptoms or relieving the condition partially or completely. The term "treat" must also be understood as encompassing the reduction of number of relapses and/or the reduction of intensity of the relapses and/or the increase of the time separating two relapses of a condition according to the present invention, in particular of skin ageing and skin photoageing.

In the context of the present invention, the term *"prevent"* or *"prevention"* denotes the reduction to a lesser degree of the risk or of the probability of occurrence of a given phenomenon, that is to say, in the present invention, skin ageing or skin photoageing. The term *"prevent"* must also be understood as encompassing the reduction to a lesser degree of the risk or of the probability of relapse of a condition according to the present invention, in particular of a chronic one.

The methods and uses according to the present invention are preferably cosmetic and non-therapeutic.

*"Skin ageing"* is a multifaceted biological process that includes two main aspects: intrinsic aging, or "chronological aging," largely governed by genetics; and extrinsic aging, primarily driven by environmental factors like solar UV exposure (also known as "photoageing").

As used herein, "*signs of ageing*" are selected from the group consisting of wrinkles; fine lines; loss of elasticity; sagging of the skin, marked microrelief of the skin, the appearance of roughness, thinning of the skin, loss of firmness; loss of radiance; and pigmentation disorders such as pigmentation spots including actinic lentigo and melasma.

As defined in the present specification, an *"individual"* is a mammal, in particular is a human. In a particular embodiment, an individual as used herein is an individual over the age of 25. More particularly, an individual is over the age of 30, more particularly is over the age of 35.

By a level of expression that is *"higher than"* a control level, it is understood in the present specification that the level of expression is significantly higher than a control level as established by a statistical test, in particular by a T-test, or that the level of expression is at least 5% higher than the control level.

By a level of expression that is *"lower than"* a control level, it is understood in the present specification that the level of expression is significantly lower than a control level as established by a statistical test, in particular by a T-test, or that the level of expression is at least 5% lower than the control level.

The terms *"sequence homology"* or *"sequence identity"* or *"homology"* or *"identity"* are used interchangeably herein. For the purpose of the invention, it is defined here that in order to determine the percentage of sequence homology or sequence identity of two amino acid sequences or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The sequence identity is the percentage of identical matches between the two sequences over the reported aligned region. A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The percent sequence identity between two amino acid sequences or between two nucleotide sequences may be determined using the Needleman and Wunsch algorithm for the alignment of two sequences. (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE.

For the purpose of the invention, the NEEDLE program from the EMBOSS package was used (version 2.8.0 or higher, EMBOSS: The European Molecular Biology Open Software Suite (2000) Rice, P. LongdenJ. And Bleasby,A. Trends in Genetics 16, (6) pp276-277, http://emboss.bioinformatics.nl/). For protein sequences EBLOSUM62 is used for the substitution matrix. For nucleotide sequence, EDNAFULL is used. The optional parameters used are a gap opening penalty of 10 and a gap extension penalty of 0.5. No end gap penalty is added. In the Output section, Yes has been indicated in response to the question "Brief identity and similarity" and "SRS pairwise" indicated as Output alignment format.

After alignment by the program NEEDLE as described above the percentage of sequence identity between a query sequence and a sequence of the invention is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labeled in the output of the program as "longest-identity".

The similarity of nucleotide and amino acid sequences, i.e. the percentage of sequence identity, can be determined via sequence alignments using several other art-known algorithms, preferably with the mathematical algorithm of Karlin and Altschul (Karlin & Altschul (1993) Proc. Natl. Acad. Sci. USA 90: 5873-5877), with hmmalign (HMMER package, http://hmmer.wustl.edu/) or with the CLUSTAL algorithm (Thompson, J. D., Higgins, D. G. & Gibson, T. J. (1994) Nucleic Acids Res. 22, 4673-80) available e.g. on https://www.ebi.ac.uk/Tools/msa/clustalo/ or the GAP program (mathematical algorithm of the University of Iowa) or the mathematical algorithm of Myers and Miller (1989 - Cabios 4: 11-17) or Clone Manager 9. Preferred parameters used are the default parameters as they are set on https://www.ebi.ac.uk/Tools/msa/clustalo/.

The grade of sequence identity (sequence matching) may be calculated using e.g. BLAST, BLAT or BlastZ (or BlastX). A similar algorithm is incorporated into the BLASTN and BLASTP programs of Altschul et al (1990) J. Mol. Biol. 215, 403-410. BLAST polynucleotide searches are performed with the BLASTN program, score = 100, word length = 12, to obtain polynucleotide sequences that are homologous to those nucleic acids which encode the relevant protein.

BLAST protein searches are performed with the BLASTP program, score = 50, word length = 3, to obtain amino acid sequences homologous to the SHC polypeptide. To obtain gapped alignments for comparative purposes, Gapped BLAST is utilized as described in Altschul et al (1997) Nucleic Acids Res. 25, 3389-3402. When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1: 154-162) or Markov random fields. When percentages of sequence identity are referred to in the present application, these percentages are calculated in relation to the full length of the longer sequence, if not specifically indicated otherwise.

In particular embodiments, % identity between two sequences is determined using CLUSTAL O (version 1.2.4).

It is to be noted that the term "a" or "an" entity refers to one or more of that entity; for example, "an antibody," is understood to represent one or more antibodies. As such, the terms "a" (or "an"), "one or more" and "at least one" can be used interchangeably herein.

Throughout this specification and embodiments, the words "have" and "comprise," or variations such as "has", "having", "comprises" or "comprising" will be understood to imply the inclusion of a stated integer or group of integers but not the exclusion of any other integer or group of integers. The words "have" and "comprise" or variations such as "has", "having", "comprises" or "comprising" will be understood to imply the inclusion of the stated element(s) (such as a composition of matter or a method step) but not the exclusion of any other elements. The term "consisting of" implies the inclusion of the stated element(s), to the exclusion of any additional elements. The term "consisting essentially of" implies the inclusion of the stated elements, and possibly other element(s) where the other element(s) do not materially affect the basic characteristic(s) of the disclosure. It is understood that the different embodiments of the disclosure using the term "comprising" or equivalent cover the embodiments where this term is replaced with "comprising only", "consisting of" or "consisting essentially of".

It is understood that wherever aspects are described herein with the language "comprising" otherwise analogous aspects described in terms of "consisting of" and/or "consisting essentially of" are also provided.

Furthermore, "and/or" where used herein is to be taken as specific disclosure of each of the two specified features or components with or without the other. Thus, the term "and/or" as used in a phrase such as "A and/or B" herein is intended to include "A and B", "A or B", "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

### Method for screening active compounds

The present invention relates to a method for screening active compounds to prevent and/or treat the signs of skin ageing, said method comprising the following steps:
(i) contacting a candidate active compound with a cell or tissue sample, in particular a skin cell or a skin tissue sample;
(ii) measuring the level of expression of miR-383, and/or PCLAF and/or TTK in the cell or tissue sample, in particular the skin cell or skin tissue sample;
(iii) comparing the level of expression of miR-383, and/or PCLAF and/or TTK measured in step (ii) with a control level; and
(iv) selecting the candidate active compound as an active compound to prevent and/or treat the signs of skin ageing when:
   a. the level of expression of miR-383 measured in step (ii) is lower than the control level, and/or
   b. the level of expression of PCLAF measured in step (ii) is higher than the control level, and/or
   c. the level of expression of TTK measured in step (ii) is higher than the control level.

In a particular embodiment, an active compound as used herein is a cosmetic active compound. In particular, a candidate active compound is a cosmetic candidate active compound.

As used herein, a cell sample may be selected from the group consisting of dermal cells from skin biopsy which express miR-383, in particular dermal fibroblasts from skin biopsy which express miR-383; epidermal keratinocytes from skin biopsy which express miR-383 ; or cell lines which express miR-383 such as endothelial cell line isolated from the vein of the umbilical cord (HUVEC), or epidermoid carcinoma cell line A431, or epithelium ovarian cancer cell line such as CAOV3, OVCAR3,SKOV3, ES2, CAOV4, OVCA429, OVCA432, OVAS, or hepatocellular carcinoma cell line such as Huh7 and HepG2 ; or cholangiocarcinoma cell line such as HuCCT1, RBE, QBC939, CCLP. Endothelial cell lines isolated from the vein of the umbilical cord (HUVEC) are known as cell lines expressing miR-383 from the publication Wang Het al., Bioengineered. 2022;13(5):13728-13738.

Epidermoid carcinoma cell line A431 is known as a cell line expressing miR-383 from the publication Liao XH et al., Cell Signal. 2015;27(11):2285-2295.

Epithelium ovarian cancer cell lines such as CAOV3, OVCAR3,SKOV3, ES2, CAOV4, OVCA429, OVCA432, OVAS are already known as cell lines expressing miR-383 from the publication Liu J et al., Biomed Pharmacother. 2016;83:1286-1294.

Hepatocellular carcinoma cell lines such as Huh7 and HepG2 are already known as cell lines expressing miR-383 from the publication Shi Y et al., Tumour Biol. 2015;36(11):8455-8463.

The said cholangiocarcinoma cell line such as HuCCT1, RBE, QBC939, CCLP is already known as a cell line expressing miR-383 from the publication Wan P et al., J Cell Biochem. 2018;119(12):9720-9729.

In a preferred embodiment, the dermal cells, or fibroblasts or epidermal keratinocytes expressing miR-383, express miR-383 either constitutively, in particular said dermal cells or fibroblasts or epidermal keratinocytes come from sun-exposed skin of donors preferentially over 70 years old or are modified to overexpress miR-383 using methods known by a skilled person.

As used herein, a tissue sample may be selected from the group consisting of skin biopsy of skin explants which express miR-383 or reconstructed or synthetic skin models which express miR-383.

In a preferred embodiment, the said skin explants comprise dermal cells, or fibroblasts or epidermal keratinocytes expressing miR-383, in particular constitutively, more particularly the said skin explants comprise dermal cells, or fibroblasts or epidermal keratinocytes expressing miR-383 that come from sun-exposed skin of donors preferentially over 70 years old.

In a preferred embodiment, the said reconstructed or synthetic skin models include dermal cells, or fibroblasts or epidermal keratinocytes expressing miR-383, in particular constitutively, more particularly the said skin explants comprise dermal cells, or fibroblasts or epidermal keratinocytes expressing miR-383 that come from sun-exposed skin of donors preferentially over 70 years old or modified to overexpress miR-383 using methods known by skilled person.

In a preferred embodiment, the cell or tissue sample is a human cell or tissue sample, in particular is a human skin cell or a human skin tissue sample.

Measuring the levels of miR-383 as used herein means measuring the RNA levels of the miR-383.

According to a particular embodiment, step (ii) of the method according to the invention may further comprise measuring the level of expression and/or the level of activity of at least one senescence biomarker chosen from the list consisting of β-galactosidase (β-gal), p16, SIRT1, p21, RB, p53 , MKI67, MMPs such as MMP1 and/or MMP3, IL-1β, TNF-α, IL-8, IL-6, IL-1α, fibronectin, collagens, laminin, GDF15, TGFβ1, IFNγ, phosphorylation of the histone variant H2AX (γH2AX) and focal assembly of checkpoint and senescence-associated heterochromatic foci (SAHF) , histone H3 trimethylated at Lys9 such as H3K9me3, histone H3 trimethylated at Lys27 such as H3K27me3, LINE-1, SADS, TAF, LMNB1, ABL1, EFNB1, BCL2L1, EFNB3, SERPINB2 ,PI3K, production of reactive oxygen species (ROS), uPAR, DDP4, NOTCH1, TNFRSF10D, NOTCH3, CD36, Oxidized vimentin, and ICAM1.

In a particular embodiment, measuring the level of expression of at least one senescence biomarker comprises measuring the mRNA levels of at least one senescence biomarker and/or measuring the protein levels of at least one senescence biomarker and/or the level. In a particular embodiment, measuring the level of activity of at least one senescence biomarker comprises measuring the enzymatic activity of at least one senescence biomarker, for example, without limitation, if the senescence biomarkers are MMPs, the activity of hydrolysis or degradation of their protein substrate such as collagen can be measured.

In a particular embodiment:
- measuring the level of expression of PCLAF comprises measuring the mRNA levels of PCLAF and/or measuring the protein levels of PCLAF; and/or
- measuring the expression of TTK comprises measuring the mRNA levels of TTK and/or measuring the protein levels of TTK.

Methods of measuring mRNA levels and of measuring protein levels are well-known in the art.

Methods of measuring mRNA levels may include, but are not limited to, reverse transcription quantitative polymerase chain reaction (RT-qPCR or qRT-PCR); RNA Sequencing (RNA-Seq), Northern Blotting; Microarray Analysis; In Situ Hybridization (ISH); Digital PCR (dPCR); Single-Cell RNA Sequencing (scRNA-Seq); Quantitative Nucleic Acid Sequence-Based Amplification (QT-NASBA); Luminex x MAP Technology; Fluorescence-Based mRNA Detection (such as FISH); and Real-Time LAMP (Loop-mediated Isothermal Amplification).

According to a particular embodiment, the mRNA levels of the miR-383, the mRNA levels of PCLAF and/or the mRNA levels of TTK are measured by RT-qPCR, RNA-Seq or dPCR, preferably by RT-qPCR.

RT-qPCR, also named qRT-PCR, is a highly sensitive method that involves converting mRNA into complementary DNA (cDNA) using reverse transcriptase, followed by quantitative PCR amplification of the cDNA. This allows for precise measurement of specific mRNA levels.

Methods of measuring protein levels may include, btu are not limited to, Western Blotting (Immunoblotting); Enzyme-Linked Immunosorbent Assay (ELISA) ; Mass Spectrometry (MS); Immunohistochemistry (IHC); Proteome Profiler Arrays; Liquid Chromatography-Mass Spectrometry (LC-MS/MS); Flow Cytometry; Surface Plasmon Resonance (SPR); Fluorescence Polarization (FP) or Fluorescence Resonance Energy Transfer (FRET).

According to a particular embodiment, the protein levels of PCLAF and/or the protein levels of TTK are measured by Western Blotting or ELISA, preferably by Western Blotting.

According to a particular embodiment, the control level in step (iv) a. is the level expression of miR-383 measured in the cell or tissue sample, in particular the skin cell or skin tissue sample, before step (i), i.e. the level of expression of miR-383 measured before the cell or tissue sample, in particular the skin cell or skin tissue sample, is put in contact with the candidate active compound.

According to a particular embodiment, the control level in step (iv) b. is the level of expression of PCLAF measured in the cell or tissue sample, in particular the skin cell or skin tissue sample, before step (i), i.e. the level of expression of PCLAF measured before the cell or tissue sample, in particular the skin cell or skin tissue sample, is put in contact with the candidate active compound.

According to a particular embodiment, the control level in step (iv) b. is the level of expression of TTK measured in the cell or tissue sample, in particular the skin cell or skin tissue sample, before step (i), i.e. the level of expression of TTK measured before the cell or tissue, in particular the skin cell or skin tissue sample, sample is put in contact with the candidate active compound.

Similarly, when the method of the invention comprises in step (ii) further measuring the level of expression and/or the level of activity of at least one senescence biomarker, different from miR-383, PCLAF and TTK, it thus also comprises appropriate steps (iii) and (iv).

In a particular embodiment, the method according to the invention is implemented *in vitro* or *ex vivo.*

Thus, in a particular embodiment, the method according to the invention may comprise a step prior to step (i) wherein a cell or tissue sample, in particular the skin cell or skin tissue sample, is obtained from an individual. Methods of obtaining a cell or tissue sample, in particular a skin cell or skin tissue sample, from an individual are well know in the art. Such methods include, but are not limited to, skin biopsies, tape stripping, suction blistering, punch biopsy, and skin scrapings. According to the nature of the cell or tissue sample, in particular of the skin sample, one skilled in the art will know to adapt the method for obtaining the sample.

### Use of a miR-383, PCLAF and/or TTK modulator

According to another aspect, the present invention relates to the use of at least one modulator that:
(a) inhibits, directly or indirectly, the expression of miR-383; and/or
(b) induces, directly or indirectly, the expression of PCLAF and/or TTK;
to prevent and/or treat the signs of skin ageing.

The invention further relates to the use of at least one modulator that:
(a) inhibits, directly or indirectly, the expression of miR-383; and/or
(b) induces, directly or indirectly, the expression of PCLAF and/or TTK;
to prevent and/or reverse dermal fibroblast senescence in the skin of an individual.

The signs of skin ageing according to the invention are defined throughout the specification.

Dermal fibroblast senescence as used herein defines the process in which fibroblasts, the cells responsible for producing extracellular matrix and collagen in the skin, undergo irreversible growth arrest due to factors like oxidative stress, DNA damage, or aging. This leads to a decline in their ability to maintain skin structure and function, contributing to skin aging and the formation of wrinkles.

Dermal fibroblast senescence can be measured by assessing markers such as β-galactosidase activity (senescence-associated β-gal), the expression of cell cycle inhibitors (e.g., p16INK4a), or the accumulation of senescence-associated secretory phenotype (SASP) factors.

Other senescence hallmarks can be assessed to monitor cellular senescence. For example, accumulation of the CDK2 inhibitor CDKN1A (also known as p21CIP1 or p21), activation of RB and p53 (encoded by TP53 in humans) or increase of MKI67 are involved in senescent cell cycle arrest. Senescent fibroblasts are characterized not only by irreversible growth arrest but they contribute also to a decline in tissue function by the enhanced production of matrix-degrading enzymes such as metalloproteinases (MMPs), proinflammatory cytokines, e.g. IL-1β, TNF-α, IL-6, and IL-8 and concomitant decreased production of growth factors collectively termed as Senescence-Associated Secretory Phenotype (SASP), characterized by a wide array of pro-inflammatory cytokines, chemokines and interleukins such as IL-8, IL-6, IL-1α or IL-1β, along with cell-growth modulators, proteases and their inhibitors, angiogenic factors and insoluble factors such as fibronectin, collagens and laminin, and other inflammatory molecules such as GDF15, TGFβ1 and IFNγ (COPPE et al., Annu Rev Pathol. 2010;5:99-118; FITSIOU et al., J Invest Dermatol. 2021;141(4s):1119-1126; WLASCHEK et al., BMB Rep. 2019;52(1):24-34; and WALDERA et al., J Invest Dermatol. 2015;135(8):1954-1968). Other senescence hallmarks include activation of the DNA damage response (DDR), including phosphorylation of the histone variant H2AX (γH2AX) and focal assembly of checkpoint and DNA repair factors (for example, T53BP1) at sites of DNA damage across the genome, nuclear changes with global loss and local gain of heterochromatin, known as senescence-associated heterochromatic foci (SAHF) and characterized by gain of histone H3 trimethylated at Lys9 (H3K9me3) and Lys 27 H3K27me3, activation of transposable elements such as long interspersed element-1 (LINE-1), senescence-associated distension of satellites (SADS), telomere-associated foci (TAF) formation, and loss of lamin B1 (LMNB1) and lamina-associated domains, upregulation of anti-apoptotic factors such as ABL1, EFNB1, BCL2L1, EFNB3, SERPINB2 and PI3K, changes in the function of mitochondria, with increase the production of reactive oxygen species (ROS), increase of some cell surface markers (senescence-associated protein and glycan receptors, such as uPAR, DDP4, NOTCH1, TNFRSF10D, NOTCH3, CD36, Oxidized vimentin, ICAM1), changes in morphology (cells become, enlarged and vacuolized, with enlarged nuclei and nuclear blebbing, leading to the formation of cytoplasmic chromatin fragments) (SURYADEVARA et al., Nat Rev Mol Cell Biol. 2024; WANG et al., Front Genet. 2018;9:247; GRUBER et al., Exp Gerontol. 2020;130:110780, GONZALEZ-GUALDA et al., Febs j. 2021;288(1):56-80; LOPEZ-OTIN et al., Cell. 2023;186(2):243-278 ; and CHIN et al., Frontiers in physiology. 2023;14:1297637).

As used herein, a modulator that directly inhibits the expression of miR-383, PCLAF or TTK in a modulator that directly targets and reduces the expression of these genes at the transcriptional or post-transcriptional level. On the contrary, a modulator that indirectly inhibits the expression of miR-383, PCLAF or TTK is a modulator that alters signaling pathways or regulatory factors, leading to a reduction in the expression of these genes.

As used herein, a modulator that directly activates the expression of miR-383, PCLAF or TTK is a modulator that directly promotes or enhances the expression of these genes at the transcriptional or post-transcriptional level. On the contrary, a modulator that indirectly activates the expression of miR-383, PCLAF or TTK is a modulator that alters signaling pathways or regulatory factors, leading to an increase in the expression of these genes.

In particular, the at least one modulator that inhibits directly or indirectly the expression of miR-383 may be an antisense oligonucleotide. In particular, the at least one modulator that inhibits directly or indirectly the expression of miR-383 may be an antisense oligonucleotide selected from the list consisting of antagomirs such as antisense microRNA oligonucleotides (AMO); and shRNAs.

Antagomirs are chemically modified oligonucleotides designed to specifically bind and inhibit the activity of microRNAs (miRNAs) by preventing their interaction with target mRNAs, thereby modulating gene expression. They are often used in research and therapeutic applications to suppress the function of specific miRNAs. An example of antagomirs are antisense microRNA oligonucleotides (AMOs). While antagomirs are typically chemically modified to enhance stability and potency, AMOs are also designed to be complementary to the miRNA of interest, thereby blocking its function.

Short hairpin RNA (shRNA) are RNA molecules that form a stem-loop structure and are used to silence gene expression by triggering RNA interference (RNAi). They are often introduced into cells to induce the degradation of specific target mRNAs, effectively reducing the expression of the corresponding gene.

According to a particular embodiment, the antisense oligonucleotide comprises a nucleic acid sequence:
- having at least 80 %, or at least 85 %, or at least 90% or at least 95 % identity sequence with the nucleic acid sequence set forth as SEQ ID NO: 1 and comprising at least a sequence having 100% sequence identity with the nucleic acid sequence set forth as SEQ ID NO: 3, or
- having at least 80 %, or at least 85 %, or at least 90% or at least 95 % identity sequence with the nucleic acid sequence set forth as SEQ ID NO: 2 and comprising at least a sequence having 100% sequence identity with the nucleic acid sequence set forth as SEQ ID NO: 4,
or a derivative thereof; or solvates thereof such as hydrates; or optical or geometric isomers or tautomers thereof; or organic or mineral base or acid salts thereof.

Solvates of such nucleic acid sequences such as hydrates; or optical and geometric isomers and tautomers thereof; or organic or mineral base or acid salts thereof are described for example in EGLI et al., Nucleic Acids Res. 2023;51(6):2529-2573, SHI et al., Chem Rev. 2024;124(10):6690-6692; ZHARKOV et al., Molecules. 2024;29(2); WAN et al., J Med Chem. 2016;59(21):9645-9667; SAHU et al., Curr Protoc Nucleic Acid Chem. 2017;69:14.13.11-14.13.15; ZENGIN et al., Org Lett. 2024;26(19):4137-4141; YU et al., Bioorg Med Chem Lett. 2023;83:129172; or in SETH et al., Natural Products In Medicinal Chemistry. Vol 60. Germany: Wiley-VCH Verlag GmbH & Co; 2014:403-440).

In a particular embodiment, the antisense oligonucleotide comprises a sequence having 100% sequence identity with the nucleic acid sequence set forth as SEQ ID NO: 1 or comprises a sequence having 100% sequence identity with the nucleic acid sequence set forth as SEQ ID NO: 2, or a derivative thereof; or solvates thereof such as hydrates; or optical or geometric isomers or tautomers thereof; or organic or mineral base or acid salts thereof.

In a particular embodiment, the antisense oligonucleotide consists of the nucleic acid sequence set forth as SEQ ID NO: 1 or of the nucleic acid sequence set forth as SEQ ID NO: 2.

According to a particular embodiment, the antisense oligonucleotide comprises a derivative nucleic acid sequence of any one of the nucleic acid sequences described above.

A derivative of a nucleic acid as used herein includes a nucleic acid sequence comprising a modification on the ribose moiety of at least one nucleotide, comprising a modification on the nucleobase of at least one nucleotide and/or comprising a modification of the backbone of the nucleic acid sequence.

According to a particular embodiment, modifications on the ribose moiety of a nucleotide include substitutions on the 2' position; modifications to the ribose ring, such as substitution of the oxygen atom and bridged nucleic acids (BNAs); modification of the ribose ring size; and ribose ring opening.

Substitutions on the 2' position include substitutions with a hydrogen atom (2'-deoxyribo, DNA backbone), a hydroxy group (2'-hydroxyl, RNA native backbone), a fluor atom (2'-F) and the isomer 2'-arabino-fluoro (2'-Ara-F), methoxy group (2'-O-Me), methoxyethyloxy group (2'-O-MOE), benzyloxy group (2'-O-benzyl), 4-pyridinylmethoxy group (2'-O-methyl-4-pyridine), 2'-O-(3-aminopropyl) (AP), 2'-O-[2-(guanidinium)ethyl] (GE), 2'-O-2-[2-(N,N-dimethylamino)ethoxy]ethyl] (DMAEOE), 2'-O-[2-(methylamino)-2-oxyethyl] (NMA).

Modifications to the ribose ring may include substitution of the oxygen atom by a carbon atom (4'-carbanucleoside), a sulfur atom (4'-thionucleoside), or a selenium atom (4'-selenonucleoside).

Modifications to the ribose ring may include bridged nucleic acids (BNAs), characterized by the presence of a covalent bridge connecting the 2'-O and 4'-C positions of the ribose moiety, and fused ring systems such as:
- locked nucleic acid (LNA), and analogs of LNA that replace the 2'-oxygen atom with a sulfur atom (2'-thio-LNA), with a carbon atom (carba-LNA or cLNA), or with a nitrogen atom possibly substituted (2'-amino-LNA); LNA could be further substituted, for example with a methyl group at 5' position (5'-Me-LNA), 3' position (3'-Me-LNA) or 6' position (6'-Me-LNA); cLNA could be further substituted, for example with a methyl group (Me-cLNA) or a methylene group (methylene-cLNA);
- constrained MOE (cMOE), 2',4'-constrained 2'-O-ethyl BNA (cEt-BNA or cEt);
- ring-expanded BNA analogs such as ethylene nucleic acid (ENA) and further derivatives, notably epi-cENA, subs-cENA, aza-ENA, oxyimino-LNA or aminooxy-LNA;
- BNA modifications such as 2'-O,4'-C-methylenoxymethylene-bridged nucleic acid (BNA^{COC}); guanidine-bridged 2',4'-BNA/LNA analog (GuNA); 2'-O,4'-C-aminomethylene-bridged nucleic acid (BNA^{NC}) and its variants BNA^{NC}[NH], BNA^{NC}[NMe], or BNA^{NC}[NBn]; and
- bicyclo-DNA (bcDNA), tricylco-DNA (tcDNA), and North and South methanocarba nucleic acids (N-MC and S-MC, respectively).

Modification of the ribose ring size may include:
- hexitol nucleic acids (HNA) and further derivatives such as altritol nucleic acid (ANA) and its epimer mannose nucleic acid (MNA), methoxy-ANA (Me-ANA), or fluoro hexitol nucleic acid (FHNA) and its epimer arabino 3'-fluoro hexitol nucleic acid (Ara-FHNA), or the 2'-deoxy-2'-fluoroarabinonucleic acid (FANA); and
- morpholino ring and Phosphorodiamidate Morpholino Oligomer (PMO):

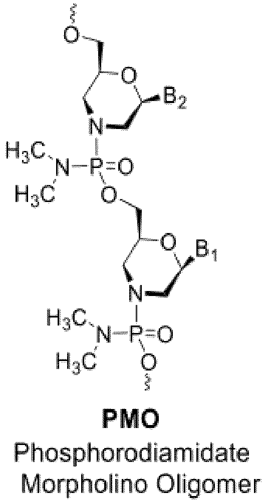

Finally, Ribose ring opening include Unlocked Nucleic Acid (UNA) and Glycol Nucleic Acid (GNA):

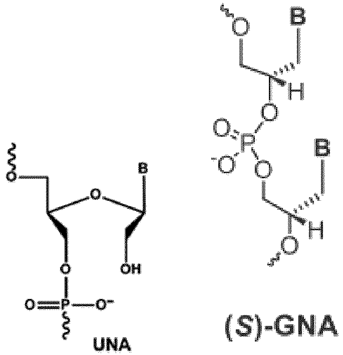

Modifications on the nucleobase of a nucleotide may include:
- Pseudouridine (Ψ), N1-Me-pseudouridine (N1-methyl-Ψ), 2-thiouridine (s2U), N6-methyladenosine (m6A), N1-methyladenosine (m1A), 7-methylguanosine (m7G), N-ethylpiperidine 7'-EAA triazole modified adenine, N-ethylpiperidine 6'-triazole modified adenine, 6'-phenylpyrrolo-cytosine (PhpC), 2',4'-difluorotoluyl ribonucleoside (rF), 5'-nitroindole, deoxyuracil (dU), 5.6-dihydro-deoxyuracil (5,6-dihydro-dU), Inosine (I), deoxyinosine (dI), isoCytosine (iso-dC) and isoGuanosine (iso-dG), N4-ethyl Cytosine (N4-Et-dC);
- 5-Substituted Pyrimidine Analogs, such as 5-Methyl-cytosine (m5C), 5-methoxy-uridine (5moU), C5-propyne T, C5-propyne C, C5-Thiazole, Phenoxazine, 5'-fluoro-2'-deoxyuridine, 2-thio-thymine, 5-triazolylphenyl-thymine, Aminoethyl-Phenoxazine 2'-deoxycytidine (AP-dC), 5-formyl deoxycytidine (5-formyl-dC), 5-carboxy deoxycytidine (5-carboxy-dC, 5-cadC), 5-methyl-deoxycytidine (5-methyl dC), 5-hydroxymethyl-deoxycytidine (hmdC, 5-hydroxymethyl dC); and
- Further purine modifications, such as: 2-aminopurine, diaminopurine, N2-aminopropylguanine, 7-propynyl-7-deaza-adenine, 7-propynyl-7-deaza-guanine, 7-deaza-deoxyadenine (7-deaza-dA), 7-deazadeoxyguanine (7-deaza-dG), 8-oxoadenine (8-oxo-dA), 8-oxoguanine (8-oxo-dG).

In a particular embodiment, modifications of the backbone of a nucleic acid sequence include modifications of the phosphodiester and phosphodiester replacement.

Modifications of the phosphodiester may include:
- Common modifications such as: Phosphorothioate (PS or PTO), Phosphorodithioate (PS2), Methylphosphonate (MP), Methoxypropylphosphonate (MOP), 5'-(E)-vinylphosphonate (E-VP), 5'-methylphosphonate (5'-MP), 5'-C-methyl with phosphate, 5'-Phosphorothioate (5'-PS);
- Modification of the 5' position: 5'-carbon elongation: 5'-homonucleoside;

o α,β-constrained nucleic acid (α,β-CNA) with an appended six-membered dioxaphosphorinane ring system; and
o Phosphoroamidate diversely substituted, for example with: *-dodecyl-containing triazinyl phosphoramidate (TPA); *-1,3-dimethylimidazolidin-2-iylidene phosphoramidate (DMI); *-di(pyrrolidine-1-yl)methylene phosphoramidate; or *-methanesulfonyl phosphoramidate.

Replacements of the phosphodiester may include:
o Amide backbone linkage (named AM1 or C3-amide): 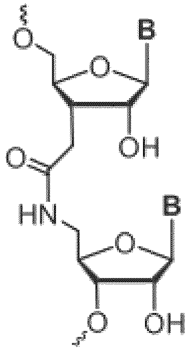
o Sulfamate and Sulfamide Backbone Linkages;
o Other modifications including: Phosphotriester diversely substituted; Boranophosphate; N3'-phosphoramidate, 3'-methylene phosphonate, formacetal, thioformacetal, Methylene(methylimino) (MMI); and
o Peptide nucleic acid (PNA):

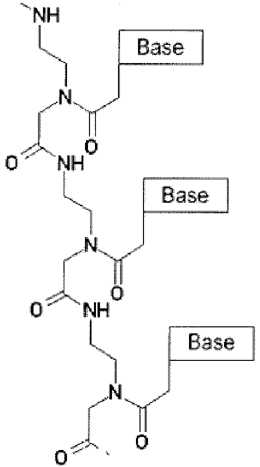

A derivative of a nucleic acid as used herein may include any combination of modifications described above.

For example, mention can be made of 2'-O-MOE-5-Me-cytidine, or LNA-Sulfamate and Sulfamide Backbone Linkages, such as described in ZENGIN et al. (Org Lett. 2024;26(19):4137-4141).

According to a particular embodiment, the antisense oligonucleotide comprises at least one 5' and/or 3' end modification.

A 5' or 3' modification may include inverted nucleotides, such as 3'-dT (3'-deoxythymidine); phosphorylation; thiophosphorylation; capping, such as capping with a methylated guanine; modification with phosphine -PH₂ ; and amino modification (such as amine -NH₂ or amino modifier such as hydrocarbon chain in C3, C6 or C12 terminated with an amine -NH₂). In a particular embodiment, the end modification is a 3'-dT.

According to a particular embodiment, the antisense oligonucleotide may be conjugated with at least one conjugate group (or tag).

Conjugates may be used to enhance stability, solubility, targeting, detection, cellular uptake, and/or functionality of the antisense oligonucleotide.

In a preferred embodiment, the antisense oligonucleotide can be encapsulated, in particular by methods as reviewed by Nele et al. (Adv. Drug Deliv. Rev. 2024, 208, 115291), Mendes et al. (Nat. Rev. Methods Primers 2022, 2(1), 24,), Bian et al. (ACS Nano 2025, 19(4), 4039-83).

In a conjugate, the antisense oligonucleotide is associated at its 5' and/or 3' end with a conjugate group (tag), by a covalent link or by base pairing with a complementary sequence, and optionally through a spacer between the oligonucleotide and the conjugate group.

The spacer according to the invention could be selected among an oligonucleotide sequence (possibly random/scramble), a hydrocarbon chain in C3, C6, or C12, PEG (polyethylene glycol), TEG (triethylene glycol), HEG (hexaethylene glycol), or polyamines. The spacer could be linear or branched, in order to attach several antisense oligonucleotides to the same linker.

The conjugate group according to the invention may be selected from the group consisting of PEG (Polyethylene Glycol), nanoparticles such as gold nanoparticles, DNA/RNA-based origami & nanostructures, peptides, transdermal peptides, cell-penetrating peptides (CPP), proteins, toxins, lipids and more particularly cholesterol, small molecule ligands such as carbohydrates, antibodies and antibody mimetics, such as nanobodies, aptamers (oligonucleotidic or peptidic) and other related mimetics such as the ones listed by X. Yu et al. (Annu. Rev. Anal. Chem. 2017. 10:293-320), these ligands being eventually specific to a particular cell biomarker.

In particular, a conjugate may be selected from polyamines; peptides; lipids such as cholesteryl, hexenyl, dodecyl, tocopheryl, squalenyl, and isoprenyl groups; PEGs (possibly with amino linker); small molecule ligands such as carbohydrates, or antibodies and antibody mimetics, in particular nanobodies or aptamers.

According to a particular embodiment, the modulator that induces directly or indirectly the expression of PCLAF is selected from the list consisting of Tosylarginine methyl ester (TAME) (CAS 1784-03-8 such as described in DUAN et al., Cancer Biol Ther. 2022;23(1):65-75); proTAME (CAS 1362911-19-0); and Cepafungin I (CAS 130743-08-7 such as described in AMATUNI et al., Cell Chem Biol. 2020;27(10):1318-1326.e1318); as well as solvates thereof such as hydrates; optical and geometric isomers and tautomers thereof; and organic or mineral base or acid salts thereof.

### Method for evaluating the efficacy of a cosmetic treatment against the signs of skin ageing

Another aspect of the present invention relates to a method for evaluating the efficacy of a cosmetic treatment against the signs of skin ageing comprising at least the steps of :
1) applying the cosmetic treatment on the skin of an individual;
2) measuring the level of expression of PCLAF and/or the level of expression of TTK in a cell or tissue sample, in particular a skin cell or skin tissue sample, of the individual;
3) comparing the level of expression of PCLAF and/or the level of expression of TTK measured in step 2) with a control level.

According to a particular embodiment, the method further comprises the additional step of:
4) after step 3), determining that the cosmetic treatment is efficient against the signs of skin ageing if the level of expression of PCLAF and/or the level of expression of TTK measured in step 2) is/are higher than the control level(s).

According to the method of the invention, a control level is a control level of expression of PCLAF and/or a control level of expression of TTK measured in a cell or tissue sample, in particular a skin cell or skin tissue sample, of the individual prior to step 1), i.e. prior to applying the cosmetic treatment. In other words, if the level of expression of PCLAF and/or the level of expression of TTK measured in step 2) is/are higher than the control level of expression measured of PCLAF and/or the control level of expression measured of TTK prior to applying the cosmetic treatment in step 1), the cosmetic treatment is considered as efficient in treating the signs of skin ageing.

In a particular embodiment,
- measuring the level of expression of PCLAF comprises measuring the mRNA levels of PCLAF and/or measuring the protein levels of PCLAF; and/or
- measuring the expression of TTK comprises measuring the mRNA levels of TTK and/or measuring the protein levels of TTK.

Methods for measuring the mRNA levels of PCLAF and/or of TTK and methods for measuring the protein levels of PCLAF and/or of TTK are provide elsewhere.

In particular, the mRNA levels of PCLAF and/or the mRNA levels of TTK are measured by RT-qPCR, RNA-Seq, or dPCR, preferably by RT-qPCR.

In particular, the protein levels of PCLAF and/or the protein levels of TTK are measured by Western Blotting or ELISA, preferably by Western Blotting.

The signs of skin ageing according to the invention are defined throughout the specification.

A cosmetic treatment as used herein may comprise one or more cosmetic active compounds, in particular one or more anti-ageing cometic active compounds. In particular, the cosmetic treatment is an anti-ageing cosmetic treatment. The cosmetic treatment may take various forms, including topical products (creams, serums, lotions), injectable treatments (botulinum toxin, dermal fillers), laser treatments, chemical peels, patches, microneedles, patches coated with microneedles, microdermabrasion, and facial masks.

### In vitro or ex vivo method of diagnosing skin ageing

A further aspect of the present invention relates to an *in vitro* or *ex vivo* method of diagnosing skin ageing in an individual comprising at least the step of measuring the level of expression of PCLAF and/or the level of expression of TTK in a cell or tissue sample, in particular a skin cell or skin tissue sample, of said individual, in particular in dermal cells, more particularly in dermal fibroblasts.

In a particular embodiment,
- measuring the level of expression of PCLAF comprises measuring the mRNA levels of PCLAF and/or measuring the protein levels of PCLAF; and/or
- measuring the expression of TTK comprises measuring the mRNA levels of TTK and/or measuring the protein levels of TTK.

Methods for measuring the mRNA levels of PCLAF and/or of TTK and methods for measuring the protein levels of PCLAF and/or of TTK are provide elsewhere.

In particular, the mRNA levels of PCLAF and/or the mRNA levels of TTK are measured by RT-qPCR, RNA-Seq, or dPCR, preferably by RT-qPCR.

In particular, the protein levels of PCLAF and/or the protein levels of TTK are measured by Western Blotting or ELISA, preferably by Western Blotting.

The signs of skin ageing according to the invention are defined throughout the specification.

In a particular embodiment, the *in vitro* or *ex vivo* method according to the invention further comprises a step of comparing the level of expression of PCLAF and/or the level of expression of TTK previously measured in the cell or tissue sample, in particular the skin cell or skin tissue sample, of said individual and comparing it with a control level.

According to a particular embodiment, a control level as used in the *in vitro* or *ex vivo* method of the invention refers to the level of expression of PCLAF and/or the level of expression of TTK measured in a cell or tissue sample, in particular a skin cell or skin tissue sample, of a reference individual of a given age. This control level will serve as an exterior reference, and will allow to compare the skin ageing of an individual with the skin ageing of the average value of the expression level of PCLAF and/or TTK in a reference group composed of individual from 18 to 25 years

According to another embodiment, a control level as used in the *in vitro* or *ex vivo* method of the invention refers to the level of expression of PCLAF and/or the level of expression of TTK measured in a cell or tissue sample, in particular a skin cell or skin tissue sample, from a non photo-exposed zone of the same individual who is undergoing the *in vitro* or *ex vivo* method of the invention at a previous time. This control level will serve as a personal reference to said individual, and will allow to study the ongoing skin ageing of the individual.

According to another embodiment, a control level as used in the *in vitro* or *ex vivo* method of the invention refers to the level of expression of PCLAF and/or the level of expression of TTK measured in a cell or tissue sample, in particular a skin cell or skin tissue sample, of the same individual who is undergoing the *in vitro* or *ex vivo* method of the invention at a previous time. This control level will serve as a personal reference to said individual, and will allow to study the ongoing skin ageing of the individual.

The specification further describes a method for preventing and/or treating the signs of skin ageing in an individual in need thereof comprising at least the step of administering to said individual at least one modulator that:
(a) inhibits, directly or indirectly, the expression of miR-383; and/or
(b) induces, directly or indirectly, the expression of PCLAF and/or TTK.

The specification further describes a method for preventing and/or reversing dermal fibroblast senescence in the skin of an individual in need thereof comprising at least the step of administering to said individual at least one modulator that:
(a) inhibits, directly or indirectly, the expression of miR-383; and/or
(b) induces, directly or indirectly, the expression of PCLAF and/or TTK.

The specification further describes a method of treating skin ageing in an individual in need thereof comprising at least the steps of:
- measuring the level of expression of PCLAF and/or the level of expression of TTK in a cell or tissue sample, in particular a skin cell or skin tissue sample, of said individual,
- comparing the level of expression of PCLAF and/or the level of expression of TTK previously measured in the cell or tissue sample, in particular the skin cell or skin tissue sample, of said individual and comparing it with a control level,
- administering a cosmetic treatment, in particular an anti-ageing cosmetic treatment, when the level of expression of PCLAF and/or the level of expression of TTK is lower than the control level.

### EXAMPLES

### Material and Methods

### 1/ Isolation of cells from skin biopsies.

To separate the epidermis and dermis, skin punch biopsies were incubated with dispase (5 U/ml) (Thermo Fisher Scientific, Uppsala, Sweden) for 14-16 hours at 4 °C. The separated epidermal and dermal sheets were then treated with trypsin-EDTA (Thermo Fisher Scientific) at 37 °C for 15 minutes to obtain a single-cell suspension. Subsequently, the cell suspensions were incubated with magnetic CD45 microbeads (Miltenyi Biotec, Bergisch Gladbach, Germany) at 4 °C for 15 minutes. The CD45neg and CD45pos cell populations were separated using magnetic activated cell sorting, following the manufacturer's instructions. The four distinct cell populations (epidermal CD45neg cells, mainly keratinocytes; epidermal CD45pos cells, representing immune cells; dermal CD45neg cells, mainly fibroblasts; dermal CD45pos cells, representing immune cells) were collected for RNA extraction and qRT-PCR.

### 2/ RNA extraction and Real-time quantitative reverse transcription PCR (qRT-PCR)

Total RNA was isolated from cells using the miRNeasy Mini Kit (Qiagen, Hilden, Germany) following the manufacturer's protocol. For the detection of miRNAs (miR-383) and RNU48, reverse transcription was conducted using the TaqMan^{™} MicroRNA Reverse Transcription Kit (Thermo Fisher Scientific), and amplification of cDNA was performed using TaqMan^{™} Universal PCR Master Mix, without AmpErase^{™} UNG (Thermo Fisher Scientific). For the detection of mRNAs (SIRT1 and PUMA1), reverse transcription was carried out using the RevertAid First Strand cDNA Synthesis Kit (Thermo Fisher Scientific), and amplification of cDNA was performed using SYBR^{™} Green PCR Master Mix (Thermo Fisher Scientific). MiRNA expression was normalized against RNU48 expression, while mRNA expression was normalized against PUM1 expression.

### 3/ Overexpression of miR-383 in human skin fibroblasts

Primary neonatal human dermal fibroblasts (HDFn) (Thermo Fisher Scientific) were cultured in Human Fibroblast Expansion Basal Medium (Thermo Fisher Scientific) supplemented with Low Serum Growth Supplement (LSGS) (Thermo Fisher Scientific), penicillin (50 units/mL) and streptomycin (50 µg/mL) (Thermo Fisher Scientific). Cells were maintained in a humidified 5% CO2 incubator at 37°C. In order to overexpress miR-383 in human dermal fibroblasts, cells were plated at 60-80% confluence and were transfected with miR-383 mimic or mimic control (Thermo Fisher Scientific) at a concentration of 1nM using Lipofectamine 3000 (Thermo Fisher Scientific). Cells were harvested 24h or 48hrs after transfection for subsequent analysis.

### 4/ β-Galactosidase staining

For SA-β-Gal staining, cells were seeded in six-well plate at a density of 1.5 ×10⁵ cells /well. SA-β-Gal was stained with the Senescence β-Galactosidase Staining Kit (Cell Signaling Technology, Leiden, The Netherlands) following the manufacturer's instructions. Briefly, after washing with phosphate-buffered saline (PBS) (twice), cells were incubated with 1X fixative solution for 10 min at room temperature. Then, cells were washed twice with PBS and incubate the mixture of X-gal and staining solution (pH 5.9-6.1) at 37°C overnight. The cells were imaged using a Leica DM IL inverted contrasting microscope.

### 6/ Protein extraction and P16 Immunoblotting

Proteins were isolated using RIPA Lysis and Extraction Buffer (Thermo Fisher Scientific), which was supplemented with cOmplete^{™} Protease Inhibitor Cocktail (Sigma-Aldrich, Stockholm, Sweden) and PhosSTOP^{™} (Sigma-Aldrich). The BCA Protein Assay was employed for colorimetric detection and quantitation of total protein (Thermo Fisher Scientific). After being denatured at 95°C for 10 minutes, 30 µg of protein per well, along with a protein ladder (Bio-Rad, Stockholm, Sweden), was loaded for sodium dodecyl sulfate-polyacrylamide gel electrophoresis (SDS-PAGE). Subsequently, the proteins were transferred onto nitrocellulose membranes. Nitrocellulose membranes were blocked with 5% milk for 1 hour, followed by an overnight incubation at 4°C with gentle shaking in the presence of the appropriate primary antibodies. Afterward, the membranes underwent three 10-minute washes with PBST and were then incubated with horseradish peroxidase-coupled isotype-specific secondary antibodies (Dako, Glostrup, Denmark) for 1 hour at room temperature. Detection of the proteins of interest was accomplished using an ECL kit (Thermo Fisher Scientific), and the resulting images were captured utilizing the ChemiDoc MP Imaging System (Bio-Rad). Primary antibodies, namely anti-P16 INK4A (#80772, Cell Signaling Technology, Leiden, The Netherlands) was used at a 1:1000 dilution in PBS containing 1% BSA. β-actin (Abcam) was utilized as a control to ensure the equivalent loading and transfer of total cellular protein.

### 7/ Genetic ablation of MIR383 in human dermal fibroblasts

The MIR383 gene was deleted using a pair of single guide RNAs (sgRNAs) targeting the two ends of its gene locus (MIR383 sgRNA#1: CACAATCACCTTCTGATCTG (SEQ ID NO: 5); MIR383 sgRNA#2: GACACTTGCTCTTTCTGACC (SEQ ID NO: 6)). For each MIR383 knockout reaction, 7×10⁵ human dermal fibroblasts were transfected with a pair of sgRNAs (120 pmol each) and 26 pmol of SpCas9 2NLS Nuclease (Synthego, CA, USA) through nucleofection using the Human Dermal Fibroblast Nucleofector^{™} Kit (Lonza, Solna, Sweden).

### 8/ Replicative senescence model

Primary Neonatal Human Dermal Fibroblasts (HDFn) were serially passaged in a humidified 5% CO2 incubator at 37°C until reaching replicative senescence (i.e. until they were no longer capable of successfully undergoing cellular proliferation). Cells were passaged once they reached approximately 80 - 90% confluency. Cells were seeded at a density of 5 x 10⁴ cells per 100mm culture dish. SA-β-Gal staining was performed on WT and MIR383KO HDFn at the end of the replicative senescence models (Day 181)

### 9/ Genetic ablation of KIAA0101/PCLAF in human dermal fibroblasts

For generating KIAA0101 knockout cells, a single guide RNA (sgRNA) targeting the 5' exon of the PCLAF/KIAA0101 gene was used, with two distinct sgRNAs designed for replicates (KIAA0101 sgRNA#1: CGAACTGACTTCCCAGCCGA (SEQ ID NO: 7); KIAA0101 sgRNA#2: AGGCACTTACAGAAAAGGTG (SEQ ID NO: 8)). For each PCLAF/KIAA0101 knockout experiment, 7×10⁵ human dermal fibroblasts were transfected with a pair of sgRNAs (120 pmol each) and 26 pmol of SpCas9 2NLS Nuclease (Synthego, CA, USA) through nucleofection using the Human Dermal Fibroblast Nucleofector^{™} Kit (Lonza, Solna, Sweden).

### 10/ 3'UTR Luciferase reporter Assay.

The 3' UTR of KIAA0101/PCLAF mRNA was amplified and inserted into the pEZX-MT05 vector (GeneCopoeia, Rockville, MD, USA) to generate a secreted Gaussia luciferase reporter for the demonstration that KIAA0101/PCLAF is a direct target of miR-383. A mutant plasmid containing a miRNA-binding site complementary to the seed sequence of miR-383 (SEQ ID NO: 9) was constructed through overlapping PCR using the following primers: KIAA0101/PCLAF Forward#1: GAATTCAGCATGTCCTTTGCAACGACTAGTCACAAATGATGAAAAAGAATAG (SEQ ID NO: 10), KIAA0101 Reverse#1: AATGCAGCTGGCACGACAGG (SEQ ID NO: 11); KIAA0101 Forward#2: CCTGTCGTGCCAGCTGCATT (SEQ ID NO: 12), KIAA0101 Reverse#2:
CTATTCTTTTTCATCATTTGTGACTAGTCGTTGCAAAGGACATGCTGAATTC (SEQ ID NO: 13). The site-directed mutagenesis was confirmed through sequencing. The wildtype or mutated KIAA0101 3' UTR constructs were transiently co-transfected with miR-383 mimics or mimic control, and luciferase activity was assessed using the Secrete-Pair Dual Luminescence Assay Kit (GeneCopoeia).

### 11/ Genetic ablation of KIAA0101/PCLAF in human dermal fibroblasts

For generating KIAA0101 knockout cells, a single guide RNA (sgRNA) targeting the 5' exon of the PCLAF/KIAA0101 gene was used, with two distinct sgRNAs designed for replicates (KIAA0101 sgRNA#1 of SEQ ID NO: 7); KIAA0101 sgRNA#2 of SEQ ID NO: 8). For each PCLAF/KIAA0101 knockout experiment, 7×105 human dermal fibroblasts were transfected with a pair of sgRNAs (120 pmol each) and 26 pmol of SpCas9 2NLS Nuclease (Synthego, CA, USA) through nucleofection using the Human Dermal Fibroblast Nucleofector^{™} Kit (Lonza, Solna, Sweden).

### 12/ Protein extraction and TTK Immunoblotting

Proteins were isolated using RIPA Lysis and Extraction Buffer (Thermo Fisher Scientific), which was supplemented with cOmplete^{™} Protease Inhibitor Cocktail (Sigma-Aldrich, Stockholm, Sweden) and PhosSTOP^{™} (Sigma-Aldrich). The BCA Protein Assay was employed for colorimetric detection and quantitation of total protein (Thermo Fisher Scientific). After being denatured at 95°C for 10 minutes, 30 µg of protein per well, along with a protein ladder (Bio-Rad, Stockholm, Sweden), was loaded for sodium dodecyl sulfate-polyacrylamide gel electrophoresis (SDS-PAGE). Subsequently, the proteins were transferred onto nitrocellulose membranes. Nitrocellulose membranes were blocked with 5% milk for 1 hour, followed by an overnight incubation at 4°C with gentle shaking in the presence of the appropriate primary antibodies. Afterward, the membranes underwent three 10-minute washes with PBST and were then incubated with horseradish peroxidase-coupled isotype-specific secondary antibodies (Dako, Glostrup, Denmark) for 1 hour at room temperature. Detection of the proteins of interest was accomplished using an ECL kit (Thermo Fisher Scientific), and the resulting images were captured utilizing the ChemiDoc MP Imaging System (Bio-Rad). Primary antibodies, namely anti-TTK1 (N1)(SC-56968, Santa Cruz Biotechnology, Heidelberg, Germany) was used at a 1:1000 dilution in PBS containing 1% BSA. β-actin (Abcam) was utilized as a control to ensure the equivalent loading and transfer of total cellular protein.

### Example 1: Expression of miR-383 in cells isolated from skin biopsies

The expression of miR-383 was analyzed in cells isolated from human skin biopsies. CD45-positive (mostly immune cells) and CD45-negative cells were sorted from epidermis and dermis of skin biopsies (see Material and Methods section part 1), and miR-383 expression was studied using qRT-PCR (see Material and Methods section part 2).

The results showed that MiR-383 expression was not detected in epidermal cells. MiR-383 was detected in dermal cells, mostly in non-immune dermal cells, attesting that in human skin, mir-383 is mostly expressed by dermal fibroblasts (see **Figure 1**).

### Example 2: Overexpression of miR-383 in human fibroblasts leads to increased senescence in human skin fibroblasts.

To assess miR-383 impact on senescence in human skin fibroblasts, senescence markers were studied in fibroblasts overexpressing miR-383 vs control fibroblasts.

Human primary dermal fibroblasts were transfected using miR-3 83 mimic or mimic control, and SA-β-Gal staining was performed 48 hours later. Compared to control fibroblasts, fibroblasts overexpressing miR-383 exhibited a marked increased β-Galactosidase staining (see **Figure 2**).

Human primary dermal fibroblasts were transfected using miR-3 83 mimic or mimic control (see Material and Methods section part 3). Proteins were extracted 24 hours later and used to detect p16 and β-actin protein expression by immunoblotting (see Material and Methods section part 6). β-actin level was used as a control to ensure the equivalent loading and transfer of total cellular protein. Compared to control fibroblasts, fibroblasts overexpressing miR-383 exhibited a significant increase in the expression of p16 protein (see **Figure 3**).

Human primary dermal fibroblasts were transfected using miR-3 83 mimic or mimic control (see Material and Methods section part 3). Total RNA was extracted 48 hours later and used to assess SIRT1 and PUMA1 using qRT-PCR (see Material and Methods section part 2). SIRT1 mRNA expression was normalized against PUM1 expression. Compared to control fibroblasts, fibroblasts overexpressing miR-383 exhibited a significant decrease in SIRT1 mRNA level (see **Figure 4**). The SIRT1 values are further provided in Table 1 below:

**Table 1**

| **SIRT1 Values** | | |
|---|---|---|
| | Mimic control | miR-383 mimic |
| Mean | 1 | 0,4376 |
| Standard deviation | 0,762 | 0,123 |
| p-value | 0,01 | |

Altogether, these results attested that miR-3 83 induced senescence in human dermal fibroblasts.

### Example 3: Genetic ablation of MIR383 gene decreased senescence in human dermal fibroblasts.

To assess the impact of decreasing the level of miR-383 on senescence in human skin fibroblasts, senescence markers were studied in fibroblasts in which MIR383 gene was deleted vs wild type control fibroblasts.

The MIR383 gene was deleted in human dermal fibroblasts using CRISPR-Cas9 technology and wild type (WT) or miR-383-deleted fibroblasts (MIR383KO) were serially passaged until reaching replicative senescence (day 181) (see Material and Methods section parts 7 and 8). At that time point, SA-β-Gal staining was performed on WT and MIR383KO fibroblasts. Compared to control wild type fibroblasts, fibroblasts in which MIR383 gene was deleted exhibited a reduced β-Galactosidase staining after replicative senescence (see Figure 5)

Next, the level of SIRT1 and PUMA1 mRNA was assessed by qRT-PCR in wild type (MiR-383 WT) or miR-383-deleted (MiR-383 KO) fibroblasts (see Material and Methods section part 7 and 2). SIRT1 mRNA expression was normalized against PUM1 expression. Compared to control wild type fibroblasts, fibroblasts in which MIR383 gene was deleted exhibited a significant increase in SIRT1 mRNA level (see **Figure 6**). The SIRT1 values are further provided in Table 2 below:

**Table 2**

| **SIRT1 Values** | | |
|---|---|---|
| | MiR-383 WT | MiR-383 KO |
| Mean | 1 | 1,7376 |
| Standard deviation | 0,389 | 0,13 |
| P value | 0,0066 | |

Finally, wild type (WT) of human dermal fibroblasts in which MIR383 gene was deleted (MIR383-/-) (see Material and Methods section part 7) were serially cultured in vitro for 100 days, until first signs of replicative senescence (see Material and Methods section part 8). Subsequently, an aliquot of cells at day 100 was re-cultured for 30 days. Compared to control wild type fibroblasts, fibroblasts in which MIR383 gene was deleted exhibited a significantly increased long term growth capacity (see **Figure 7**).

Altogether, these results attested that genetic ablation of MIR383 gene decreased senescence in human dermal fibroblasts.

### Example 4: KIAA0101/PCLAF is a direct target of miR-383 and inhibits fibroblast senescence

First, the inventors showed that KIAA0101/PCLAF mRNA is a direct target for miR-383 by using 3'UTR-Luciferase reporter vector co-transfected with miR-383 mimic or mimic control.

A Luciferase reporter vector containing the wildtype or mutated 3' UTR of KIAA0101/PCLAF mRNA (mutation in the seed sequence of miR-383) was co-transfected with miR-383 mimics or mimic control, and luciferase activity was assessed. A decrease of luciferase activity when using miR-383 mimic and wildtype KIAA0101/PCLAF 3' UTR construct indicated that miR-383 can inhibit the expression of mRNA via its 3'UTR (see Material and Methods section part 10). The use of the mutated KIAA0101/PCLAF 3' UTR and miR-383 mimic reduced this inhibition, showing that it's through binding to its seed sequence in the 3'UTR of KIAA0101/PCLAF mRNA that miR-383 exerts its inhibitory effect on KIAA0101/PCLAF.

Indeed, the luciferase activity was decreased when miR-383 mimic and 3'UTR of KIAA0101/PCLAF were co-transfected, compared to mimic control, attesting that miR-383 is able to bind to KIAA0101/PCLAF 3'UTR sequence and thus inhibit luciferase mRNA expression. If 3'UTR of KIAA0101/PCLAF was mutated in the seed sequence of miR-383 binding, the luciferase activity was not decreased compared to mimic control, attesting that the binding region of miR-383 was crucial for miR-383 to exert its inhibitory effect on luciferase reporter expression (see **Figure 8**).

Altogether these results show that miR-383 can bind to its binding region in the 3'UTR of KIAA0101/PCLAF and inhibit its expression, showing that KIAA0101/PCLAF is a direct target for miR-383. Thus, senescence impact of miR-383 could be mediated via its direct inhibitory effect on KIAA0101/PCLAF.

Next, the KIAA0101/PCLAF gene was deleted in human dermal fibroblasts using CRISPR-Cas9 technology (see Material and Methods section part 11). Wild type (WT) or KIAA0101/PCLAF-deleted fibroblasts (sgRNA#1 and sgRNA#2) were used for SA-β-Gal staining (see Material and Methods section part 4).

A clear increase in SA-β-Gal staining was observed in KIAA0101/PCLAF-deleted fibroblasts, compared to wild type fibroblasts (see **Figure 9**).

Thus, the inventors demonstrated that genetic ablation of KIAA0101/PCLAF in fibroblasts leads to increased B-galactosidase staining showing here that KIAA0101/PCLAF is able to inhibit fibroblast senescence.

### Example 5: TTK1 is a target for miR-383

TTK1 mRNA is a predicted target for miR-383 by Targetscan, i.e. it contains in its 3'UTR a sequence on which miR-383 could bind.

Human primary dermal fibroblasts were transfected using miR-3 83 mimic or mimic control (see Material and Methods section part 3). Proteins were extracted 24 hours later and used to detect TTK1.

The inventors showed that overexpression of miR-383 in human dermal fibroblasts lead to down-regulation of TTK1 protein expression (see **Figure 10A** and **Figure 10B**).

This data showed that TTK1 is a target mRNA for miR-383, i.e. it may be a direct target for miR-383, or it is an indirect target, which can be part of the miR-383 mode of action to inhibit fibroblast senescence.

SEQUENCE LISTING

SEQ ID NO: 1: antisense oligonucleotide RNA nucleic acid sequence

AGCCACAAUCACCUUCUGAUCU

SEQ ID NO: 2: antisense oligonucleotide DNA nucleic acid sequence

AGCCACAATCACCTTCTGATCT

SEQ ID NO: 3: RNA seed sequence of the antisense oligonucleotide nucleic acid sequence

CUGAUC

SEQ ID NO: 4: DNA seed sequence of the antisense oligonucleotide nucleic acid sequence

CTGATC

SEQ ID NO: 5: MIR383 sgRNA#1 nucleic acid sequence

CACAATCACCTTCTGATCTG

SEQ ID NO: 6: MIR383 sgRNA#2 nucleic acid sequence

GACACTTGCTCTTTCTGACC

SEQ ID NO: 7: KIAA0101 sgRNA#1 nucleic acid sequence

CGAACTGACTTCCCAGCCGA

SEQ ID NO: 8: KIAA0101 sgRNA#2 nucleic acid sequence

AGGCACTTACAGAAAAGGTG

SEQ ID NO: 9: seed nucleic acid sequence of miR-383

GAUCAG

SEQ ID NO: 10: KIAA0101/PCLAF Forward#1 primer nucleic acid sequence

GAATTCAGCATGTCCTTTGCAACGACTAGTCACAAATGATGAAAAAGAATAG

SEQ ID NO: 11: KIAA0101/PCLAF Reverse#1 primer nucleic acid sequence

AATGCAGCTGGCACGACAGG

SEQ ID NO: 12: KIAA0101/PCLAF Forward#2 primer nucleic acid sequence

CCTGTCGTGCCAGCTGCATT

SEQ ID NO: 13: KIAA0101/PCLAF Reverse#2 primer nucleic acid sequence

CTATTCTTTTTCATCATTTGTGACTAGTCGTTGCAAAGGACATGCTGAATTC

## Claims

1. Method for screening active compounds to prevent and/or treat the signs of skin ageing, said method comprising the following steps:
**(i)** contacting a candidate active compound with a cell or tissue sample, in particular the skin cell or skin tissue sample;
**(ii)** measuring the level of expression of miR-383, and/or PCLAF and/or TTK in the cell or tissue sample, in particular the skin cell or skin tissue sample;
**(iii)** comparing the level of expression of miR-383, and/or PCLAF and/or TTK measured in step (ii) with a control level; and
**(iv)** selecting the candidate active compound as an active compound to prevent and/or treat the signs of skin ageing when:
**a.** the level of expression of miR-383 measured in step (ii) is lower than the control level, and/or
**b.** the level of expression of PCLAF measured in step (ii) is higher than the control level, and/or
**c.** the level of expression of TTK measured in step (ii) is higher than the control level.

2. The method according to claim 1, wherein:
- measuring the level of expression of PCLAF comprises measuring the mRNA levels of PCLAF and/or measuring the protein levels of PCLAF; and/or
- measuring the expression of TTK comprises measuring the mRNA levels of TTK and/or measuring the protein levels of TTK.

3. The method according to claim 1 or 2, wherein the control level in step **(iv) a.** is the level expression of miR-383 measured in the cell or tissue sample, in particular the skin cell or skin tissue sample, before step (i); the control level in step **(iv) b.** is the level of expression of PCLAF measured in the cell or tissue sample, in particular the skin cell or skin tissue sample, before step (i) and/or the control level in step **(iv) c.** is the level of expression of TTK measured in the cell or tissue sample, in particular the skin cell or skin tissue sample, before step (i).

4. The method according to any one of claims 1 to 3, wherein the method is implemented *in vitro* or *ex vivo.*

5. **Use of at least one modulator** that:
(a) inhibits, directly or indirectly, the expression of miR-383; and/or
(b) induces, directly or indirectly, the expression of PCLAF and/or TTK;
to prevent and/or treat the signs of skin ageing.

6. **Use of at least one modulator** that:
(a) inhibits, directly or indirectly, the expression of miR-383; and/or
(b) induces, directly or indirectly, the expression of PCLAF and/or TTK;
to prevent and/or reverse dermal fibroblast senescence in the skin of an individual.

7. The use according to claim 5 or 6, wherein the at least one modulator that inhibits directly or indirectly the expression of miR-383 is an antisense oligonucleotide, in particular is an antisense oligonucleotide selected from the list consisting of antagomirs such as antisense microRNA oligonucleotides (AMO); and shRNAs.

8. The use according to claim 7, wherein the antisense oligonucleotide comprises a nucleic acid sequence:
- having at least 80 %, or at least 85 %, or at least 90% or at least 95 % identity sequence with the nucleic acid sequence set forth as SEQ ID NO: 1 **and** comprising at least a sequence having 100% sequence identity with the nucleic acid sequence set forth as SEQ ID NO: 3, or
- having at least 80 %, or at least 85 %, or at least 90% or at least 95 % identity sequence with the nucleic acid sequence set forth as SEQ ID NO: 2 **and** comprising at least a sequence having 100% sequence identity with the nucleic acid sequence set forth as SEQ ID NO: 4,
or a derivative thereof; or solvates thereof such as hydrates; or optical or geometric isomers or tautomers thereof; or organic or mineral base or acid salts thereof.

9. The use according to claim 7 or 8, wherein the antisense oligonucleotide comprises a sequence having 100% sequence identity with the nucleic acid sequence set forth as SEQ ID NO: 1 or comprises a sequence having 100% sequence identity with the nucleic acid sequence set forth as SEQ ID NO: 2, or a derivative thereof; or solvates thereof such as hydrates; or optical or geometric isomers or tautomers thereof; or organic or mineral base or acid salts thereof,
and in particular consists of the nucleic acid sequence set forth as SEQ ID NO: 1 or of the nucleic acid sequence set forth as SEQ ID NO: 2.

10. The use according to claim 5 or 6, wherein the modulator that induces directly or indirectly the expression of PCLAF is selected from the list consisting of Tosylarginine methyl ester (TAME); proTAME; and Cepafungin I; as well as solvates thereof such as hydrates; optical and geometric isomers; tautomers thereof; and organic or mineral base or acid salts thereof.

11. **Method for evaluating the efficacy of a cosmetic treatment against the signs of skin ageing** comprising at least the steps of :
1) applying the cosmetic treatment on the skin of an individual;
2) measuring the level of expression of PCLAF and/or the level of expression of TTK in a cell or tissue sample, in particular the skin cell or skin tissue sample, of the individual;
3) comparing the level of expression of PCLAF and/or the level of expression of TTK measured in step 2) with a control level.

12. The method according to claim 11, further comprising the additional step of:
4) after step 3), determining that the cosmetic treatment is efficient against the signs of skin ageing if the level of expression of PCLAF and/or the level of expression of TTK measured in step 2) is/are higher than the control level(s).

13. The method according to any one of claims 1 to 4, the use according to any one of claims 5 and 7 to 10 or the method according to claim 11 or 12, wherein the signs of skin ageing are selected from the group consisting of wrinkles; fine lines; loss of elasticity; sagging of the skin, marked microrelief of the skin, the appearance of roughness, thinning of the skin, loss of firmness; loss of radiance; and pigmentation disorders such as pigmentation spots including actinic lentigo and melasma.

14. ***In vitro* or *ex vivo* method of diagnosing skin ageing** in an individual comprising at least the step of measuring the level of expression of PCLAF and/or the level of expression of TTK in a cell or tissue sample, in particular the skin cell or skin tissue sample, of said individual, in particular in dermal cells, more particularly in dermal fibroblasts.

15. The method according to any one of claims 11 to 13 or the *in vitro* or *ex vivo* method of claim 14, wherein measuring the level of expression of PCLAF includes measuring the mRNA levels of PCLAF and/or the protein levels of PCLAF and/or wherein measuring the level of expression of TTK includes measuring the mRNA levels of TTK and/or the protein levels of TTK.
